# EUROPEAN PATENT APPLICATION

(11) **EP 4 449 985 A1**
(43) Date of publication of application: **23.10.2024**
(21) Application number: 23742975.8
(22) Date of filing: 19.01.2023
(51) Int. Cl.: A61B 5/08, A61B 5/11

(54) **AUDIO DETECTION METHOD, AND ELECTRONIC DEVICE**

(30) Priority: 21.01.2022 CN 202210073455
(71) Applicant: Huawei Technologies Co., Ltd., Shenzhen, Guangdong 518129 (CN)
(72) Inventor: FANG, Yufeng, Shenzhen, Guangdong 518129 (CN); LI, Jing, Shenzhen, Guangdong 518129 (CN); HUANG, Jiejing, Shenzhen, Guangdong 518129 (CN); CHEN, Wenjuan, Shenzhen, Guangdong 518129 (CN)
(74) Representative: Körber, Martin Hans
(86) International application number: PCT/CN2023/073167
(87) International publication number: WO 2023/138660

(57) **Abstract**

An audio detection method and an electronic device (100) are provided. The method is applied to the electronic device (100). The method includes: acquiring first audio, and identifying a type of the first audio (S1301); acquiring a first signal by using one or more sensors, where the electronic device (100) includes the one or more sensors (S1302); determining a first time interval based on the first audio when the first audio is identified as a set type, where the first time interval includes a start time and an end time (S1303); determining a second signal based on the first time interval and the first signal (S1304); and displaying first information if the second signal matches the first audio (S1305). According to the method, a matching relationship between audio acquired by the electronic device (100) and another signal can be detected, so that subsequent processing can be performed based on the matching relationship.

## Description

### CROSS-REFERENCE TO RELATED APPLICATIONS

This application claims priority to Chinese Patent Application No. 202210073455.0, filed with the China National Intellectual Property Administration on January 21, 2022 and entitled "AUDIO DETECTION METHOD AND ELECTRONIC DEVICE", which is incorporated herein by reference in its entirety.

### TECHNICAL FIELD

This application relates to the field of electronic device technologies, and in particular, to an audio detection method and an electronic device.

### BACKGROUND

Respiratory diseases are common and frequently occurring diseases, and include some infectious diseases. Therefore, it is necessary to detect some respiratory diseases conveniently and efficiently.

Currently, conventional methods for detecting respiratory diseases mainly include doctor consultation, chest imaging examination, sputum culture, and the like. These methods usually require manual intervention, and are costly and time-consuming. As a result, detection efficiency is very low. This gives rise to more studies on detection of respiratory diseases (for example, asthma, chronic obstructive pulmonary disease, and novel coronavirus disease) based on a cough sound, a breath sound, or the like. Most respiratory diseases have similar symptoms such as cough, fever, and dyspnea. Therefore, an intelligent algorithm can be used to perform analysis based on these symptom characteristics to detect whether there is a respiratory disease.

Currently, a method for detecting a respiratory disease based on a cough sound is mainly applied to a wearable device. The wearable device detects a cough sound of a user in real time, so that a risk of the user suffering from the respiratory disease can be quickly analyzed. However, in the method, when there are a plurality of users in an environment in which the wearable device is located (for example, a scenario in which the user wearing the wearable device is in a public environment), in a process of performing disease risk analysis based on the cough sound detected in real time, the wearable device may mistakenly use a cough sound of another user as the cough sound of the user wearing the wearable device for detection. This may cause false detection and lead to an error in a detection result of the user wearing the wearable device.

### SUMMARY

This application provides an audio detection method and an electronic device, to detect a matching relationship between audio acquired by the electronic device and another signal, so that subsequent processing can be performed based on the matching relationship.

According to a first aspect, this application provides an audio detection method, applied to an electronic device. The method includes: acquiring first audio, and identifying a type of the first audio; acquiring a first signal by using one or more sensors, where the electronic device includes the one or more sensors; determining a first time interval based on the first audio when the first audio is identified as a set type, where the first time interval includes a start time and an end time; determining a second signal based on the first time interval and the first signal; and displaying first information if the second signal matches the first audio.

In the method, for audio of the set type, the electronic device can implement matching between the audio and another signal during generation of the audio, to determine a matching relationship between the audio and the another signal, and corresponding subsequent processing can be performed when the audio matches the another signal. Based on this, the method can implement matching between specific audio and another signal, so that subsequent processing can be performed based on a matching relationship between the audio and the another signal, for example, further implement matching between the audio and an object corresponding to the another signal. For example, when the another signal is a signal measured for a user, based on the method, matching between the audio and the user can be implemented through matching between the audio and the another signal. In this way, a correspondence between the audio and the user can be determined, so that subsequent processing is performed based on the correspondence, thereby improving accuracy of a subsequent processing process. For example, after it is determined that the audio matches the user, a disease risk of the user may be detected based on the audio, to improve detection accuracy and reduce an error.

In a possible design, the method further includes: displaying second information if the second signal does not match the first audio.

In the method, processing performed by the electronic device when the audio matches the signal is different from that performed by the electronic device when the audio does not match the signal. Therefore, through matching between the audio and the signal, the electronic device may perform corresponding processing based on the determined matching relationship, to ensure smooth execution of the subsequent processing process, and to improve accuracy and flexibility of the subsequent processing process to some extent.

In a possible design, before the determining a first time interval based on the first audio, the method further includes: determining a first risk parameter based on the first audio. The first risk parameter is greater than or equal to a set threshold. The first risk parameter indicates a disease risk corresponding to the first audio.

In the method, the electronic device may determine a corresponding risk parameter based on the audio. When the risk parameter is relatively large, risk response processing is necessary. Therefore, the electronic device may continue to perform a subsequent matching process. When the risk parameter is relatively small, risk response processing may not be performed. Therefore, the electronic device may not perform the subsequent matching process, and may start a next audio acquiring and processing process. Based on this, the electronic device may flexibly switch a processing manner based on a value of the risk parameter, and improve accuracy of the subsequent processing process.

In a possible design, the first information indicates that the first audio is from a first user and/or that the first user has a disease risk, where the first user is a user wearing the electronic device. The second information indicates that the first audio is not from the first user and/or that there is an infection risk in an environment around the first user.

In the method, the second signal may be a signal acquired for the first user. When the first audio matches the second signal, it may be determined that the first audio also matches the first user. When the first audio does not match the second signal, it may be determined that the first audio does not match the first user either. Further, when the first risk parameter is relatively large, if the first audio matches the second signal, it may be determined that the first user has a relatively high disease risk; or if the first audio does not match the second signal, it may be determined that there is an infection risk in the environment around the first user. Based on this, the electronic device may provide a more comprehensive and accurate risk prompt for the first user, so that the first user actively takes a corresponding measure to reduce a risk, thereby improving user experience.

In a possible design, the determining a first risk parameter based on the first audio includes: extracting characteristic data from the first audio, where the characteristic data represents a time-domain characteristic and/or a frequency-domain characteristic of the first audio; and determining the first risk parameter based on a set disease screening model and the characteristic data, where the disease screening model indicates a relationship between a time-domain characteristic and/or a frequency-domain characteristic of audio and a risk parameter corresponding to the audio.

In the method, relatively comprehensive audio analysis can be performed based on a characteristic such as the time-domain characteristic and the frequency-domain characteristic of the audio, and accuracy of the audio analysis can be improved by using a model, so that a risk parameter corresponding to the first audio is more accurately determined, thereby improving accuracy of the audio analysis.

In a possible design, before the displaying first information if the second signal matches the first audio, the method further includes: determining, based on the first audio, a first action corresponding to the first audio, and determining, based on the second signal, a second action corresponding to the second signal. That the second signal matches the first audio includes: A type of the second action corresponding to the second signal is the same as a type of the first action corresponding to the first audio.

In the method, the audio and the another signal are different types of information. However, if the audio and the another signal are from a same object, some characteristics of the object may be reflected. Therefore, it may be determined whether the audio matches the another signal based on whether the audio and the another signal correspond to a same characteristic. In the method, the same characteristic may be an action type. In this case, matching between the audio and the signal may be implemented by comparing whether action types corresponding to the audio and the signal are the same. For example, when both the audio and the signal are from the user, the action may be an action performed by the user. When action types corresponding to the audio and the signal are the same, it may be determined that the audio and the signal are from a same user.

In a possible design, the determining, based on the second signal, a second action corresponding to the second signal includes: determining the second action and a confidence level of the second action based on a set action recognition model and the second signal, where the action recognition model represents a relationship between the second signal and both the second action and the confidence level, and the confidence level represents recognition accuracy of the action recognition model; and determining that the confidence level is greater than or equal to a set first threshold; or determining that the confidence level is greater than or equal to a set second threshold and is less than or equal to a set third threshold; displaying first indication information, where the first indication information indicates to confirm whether the second action is performed; and receiving second indication information, where the second indication information indicates a confirmation that the second action is performed.

In the method, accuracy of action recognition can be improved by using the model, so that the action corresponding to the second signal and the type of the action can be more accurately determined, thereby improving accuracy during matching. In addition, the first indication information may be sent to the user, and the second indication information may be from the user. The electronic device may flexibly switch the subsequent processing process based on an accuracy parameter, namely, a confidence level, of the action recognition model, to ensure accuracy of a final matching result. When the confidence level is relatively small, it may be considered that the first audio does not match the second signal. Therefore, the electronic device may not perform the subsequent matching process, and may start a next audio acquiring and processing process. When the confidence level is within a range of an intermediate value, additional confirmation may be performed, to determine, based on a confirmation result, whether to perform the subsequent matching process, thereby ensuring processing accuracy. Based on this, the electronic device may flexibly switch a processing manner based on a value of the confidence level, thereby improving accuracy of the subsequent matching process.

In a possible design, the second signal includes at least one of the following: a signal acquired by using an acceleration sensor; a signal acquired by using a gyroscope sensor; and a signal acquired by using a photoelectric sensor.

In the method, the signal measured by the acceleration sensor or the gyroscope may represent a motion and posture characteristic of the user, and the signal measured by the photoelectric sensor may represent a physiological characteristic of the user. All these signals may reflect a related characteristic when the user performs an action. Therefore, the action corresponding to the second signal and the type of the action can be accurately determined based on these signals, thereby improving accuracy during audio matching.

In a possible design, the set type includes at least one of the following: a cough sound, a breath sound, a sneeze sound, and a joint popping sound.

In the method, a disease risk of the user may be predicted based on a type of audio, such as a cough sound or a breath sound made by the user. Therefore, through matching between this type of audio and the another signal, an object that may have a disease risk or have an infection risk may be more accurately determined with reference to a matching result and a prediction result of the disease risk, thereby further responding to prompts and improving user experience.

According to a second aspect, this application provides an audio detection method, applied to a first electronic device. The method includes: acquiring first audio, and identifying a type of the first audio; determining a first time interval based on the first audio when the first audio is identified as a set type, where the first time interval includes a start time and an end time; sending request information to a second electronic device, where the request information is used to request to obtain a signal corresponding to the first time interval; receiving a first signal from the second electronic device, where the first signal is acquired by the second electronic device by using one or more sensors, where the second electronic device includes the one or more sensors; and displaying first information if the first signal matches the first audio.

In a possible design, the method further includes: displaying second information if the first signal does not match the first audio.

In a possible design, before the determining a first time interval based on the first audio, the method further includes: determining a first risk parameter based on the first audio. The first risk parameter is greater than or equal to a set threshold. The first risk parameter indicates a disease risk corresponding to the first audio.

In a possible design, the first signal is a signal acquired by the second electronic device within the first time interval.

In a possible design, the first information indicates that the first audio is from a first user and/or that the first user has a disease risk, where the first user is a user wearing the second electronic device. The second information indicates that the first audio is not from the first user and/or that there is an infection risk in an environment around the first user.

In a possible design, the determining a first risk parameter based on the first audio includes: extracting characteristic data from the first audio, where the characteristic data represents a time-domain characteristic and/or a frequency-domain characteristic of the first audio; and determining the first risk parameter based on a set disease screening model and the characteristic data, where the disease screening model indicates a relationship between a time-domain characteristic and/or a frequency-domain characteristic of audio and a risk parameter corresponding to the audio.

In a possible design, before the displaying first information if the first signal matches the first audio, the method further includes: determining, based on the first audio, a first action corresponding to the first audio, and determining, based on the first signal, a second action corresponding to the first signal. That the first signal matches the first audio includes: A type of the second action corresponding to the first signal is the same as a type of the first action corresponding to the first audio.

In a possible design, the determining, based on the first signal, a second action corresponding to the first signal includes: determining the second action and a confidence level of the second action based on a set action recognition model and the first signal, where the action recognition model represents a relationship between the first signal and both the second action and the confidence level, and the confidence level represents recognition accuracy of the action recognition model; and determining that the confidence level is greater than or equal to a set first threshold; or determining that the confidence level is greater than or equal to a set second threshold and is less than or equal to a set third threshold; displaying first indication information, where the first indication information indicates to confirm whether the second action is performed; and receiving second indication information, where the second indication information indicates a confirmation that the second action is performed.

In a possible design, the first signal includes at least one of the following: a signal acquired by using an acceleration sensor; a signal acquired by using a gyroscope sensor; and a signal acquired by using a photoelectric sensor.

In a possible design, the set type includes at least one of the following: a cough sound, a breath sound, a sneeze sound, and a joint popping sound.

According to a third aspect, this application provides an audio detection method, applied to a system including a first electronic device and a second electronic device. The method includes: The first electronic device acquires first audio and identifies a type of the first audio. The second electronic device acquires a first signal by using one or more sensors, where the second electronic device includes the one or more sensors. The first electronic device determines a first time interval based on the first audio when the first audio is identified as a set type, where the first time interval includes a start time and an end time. The first electronic device sends request information to a second electronic device, where the request information is used to request to obtain a signal corresponding to the first time interval. The second electronic device determines the first time interval based on the received request information, and determines a second signal based on the first time interval and the first signal. The second electronic device sends the second signal to the first electronic device. The first electronic device displays first information if it is determined that the received second signal matches the first audio.

In a possible design, the method further includes: The first electronic device displays second information if it is determined that the second signal does not match the first audio.

In a possible design, before the first electronic device determines the first time interval based on the first audio, the method further includes: The first electronic device determines a first risk parameter based on the first audio. The first risk parameter is greater than or equal to a set threshold. The first risk parameter indicates a disease risk corresponding to the first audio.

In a possible design, the first information indicates that the first audio is from a first user and/or that the first user has a disease risk, where the first user is a user wearing the second electronic device. The second information indicates that the first audio is not from the first user and/or that there is an infection risk in an environment around the first user.

In a possible design, that the first electronic device determines a first risk parameter based on the first audio includes: The first electronic device extracts characteristic data from the first audio, where the characteristic data represents a time-domain characteristic and/or a frequency-domain characteristic of the first audio. The first electronic device determines the first risk parameter based on a set disease screening model and the characteristic data, where the disease screening model indicates a relationship between a time-domain characteristic and/or a frequency-domain characteristic of audio and a risk parameter corresponding to the audio.

In a possible design, before the first electronic device displays the first information if it is determined that the second signal matches the first audio, the method further includes: The first electronic device determines, based on the first audio, a first action corresponding to the first audio, and determines, based on the second signal, a second action corresponding to the second signal. That the second signal matches the first audio includes: A type of the second action corresponding to the second signal is the same as a type of the first action corresponding to the first audio.

In a possible design, that the first electronic device determines, based on the second signal, a second action corresponding to the second signal includes: The first electronic device determines the second action and a confidence level of the second action based on a set action recognition model and the second signal, where the action recognition model represents a relationship between the second signal and both the second action and the confidence level, and the confidence level represents recognition accuracy of the action recognition model. The first electronic device determines that the confidence level is greater than or equal to a set first threshold. Alternatively, the first electronic device determines that the confidence level is greater than or equal to a set second threshold and is less than or equal to a set third threshold; displays first indication information, where the first indication information indicates to confirm whether the second action is performed; and receives second indication information, where the second indication information indicates a confirmation that the second action is performed.

In a possible design, the second signal includes at least one of the following: a signal acquired by using an acceleration sensor; a signal acquired by using a gyroscope sensor; and a signal acquired by using a photoelectric sensor.

In a possible design, the set type includes at least one of the following: a cough sound, a breath sound, a sneeze sound, and a joint popping sound.

According to a fourth aspect, this application provides a system. The system includes the first electronic device and the second electronic device according to the third aspect.

According to a fifth aspect, this application provides an electronic device. The electronic device includes a display, a memory, and one or more processors. The memory is configured to store computer program code, and the computer program code includes computer instructions. When the computer instructions are executed by one or more processors, the electronic device is enabled to perform the method according to any one of the first aspect or the possible designs of the first aspect, or perform the method according to any one of the second aspect or the possible designs of the second aspect, or perform the method performed by the first electronic device or the second electronic device in any one of the third aspect or the possible designs of the third aspect.

According to a sixth aspect, this application provides a computer-readable storage medium. The computer-readable storage medium stores a computer program. When the computer program is run on a computer, the computer is enabled to perform the method according to any one of the first aspect or the possible designs of the first aspect, or perform the method according to any one of the second aspect or the possible designs of the second aspect, or perform the method performed by the first electronic device or the second electronic device in any one of the third aspect or the possible designs of the third aspect.

According to a seventh aspect, this application provides a computer program product. The computer program product includes a computer program or instructions. When the computer program or the instructions are run on a computer, the computer is enabled to perform the method according to any one of the first aspect or the possible designs of the first aspect, or perform the method according to any one of the second aspect or the possible designs of the second aspect, or perform the method performed by the first electronic device or the second electronic device in any one of the third aspect or the possible designs of the third aspect.

For beneficial effects of the second aspect to the seventh aspect, refer to descriptions of beneficial effects of the first aspect. Details are not described herein again.

### BRIEF DESCRIPTION OF DRAWINGS

FIG. 1 is a diagram of a hardware architecture of an electronic device according to an embodiment of this application;
FIG. 2 is a diagram of a software architecture of an electronic device according to an embodiment of this application;
FIG. 3 is a diagram of an audio detection method according to an embodiment of this application;
FIG. 4 is a diagram of an interface for displaying prompt information by a mobile phone according to an embodiment of this application;
FIG. 5 is a diagram of another interface for displaying prompt information by a mobile phone according to an embodiment of this application;
FIG. 6 is a diagram of still another interface for displaying prompt information by a mobile phone according to an embodiment of this application;
FIG. 7 is a diagram of yet another interface for displaying prompt information by a mobile phone according to an embodiment of this application;
FIG. 8A and FIG. 8B are a schematic flowchart of a method for detecting a risk of respiratory tract infection of a user according to an embodiment of this application;
FIG. 9 is a diagram of a function control interface of a smartwatch according to an embodiment of this application;
FIG. 10 is a diagram of an interface for displaying prompt information of a disease risk by a smartwatch according to an embodiment of this application;
FIG. 11 is a diagram of another interface for displaying prompt information of a disease risk by a smartwatch according to an embodiment of this application;
FIG. 12 is a diagram of an interface for a prompt interface of a smartwatch according to an embodiment of this application;
FIG. 13 is a diagram of an audio detection method according to an embodiment of this application;
FIG. 14 is a diagram of an audio detection method according to an embodiment of this application; and
FIG. 15 is a diagram of a structure of an electronic device according to an embodiment of this application.

### DESCRIPTION OF EMBODIMENTS

To make objectives, technical solutions, and advantages of embodiments of this application clearer, the following further describes embodiments of this application in detail with reference to the accompanying drawings. The terms "first" and "second" below in description of embodiments of this application are merely used for a description purpose, and shall not be understood as an indication or implication of relative importance or implicit indication of a quantity of indicated technical features. Therefore, a feature defined with "first" or "second" may explicitly indicate or implicitly include one or more such features.

For ease of understanding, concepts related to this application are described as examples for reference.

An electronic device may be a device having a wireless connection function. In some embodiments of this application, the electronic device may further have an audio detection (sound detection) function and/or a sensing function. In embodiments of this application, audio may also be referred to as a sound.

In some embodiments of this application, the electronic device may be a portable device, for example, a mobile phone, a tablet computer, a wearable device (for example, a watch, a band, a helmet, or a headset) with a wireless communication function, an in-vehicle terminal device, an augmented reality (augmented reality, AR)/virtual reality (virtual reality, VR) device, a notebook computer, an ultra-mobile personal computer (ultra-mobile personal computer, UMPC), a netbook, a personal digital assistant (personal digital assistant, PDA), a smart home device (for example, a smart television or a smart speaker), a smart robot, a workshop device, a wireless terminal in self driving (Self Driving), a wireless terminal in remote medical surgery (Remote Medical Surgery), a wireless terminal in a smart grid (Smart Grid), a wireless terminal in transportation safety (Transportation Safety), a wireless terminal in a smart city (Smart City), a wireless terminal in a smart home (Smart Home), or a flight device (for example, a smart robot, a hot air balloon, an uncrewed aerial vehicle, or an aircraft).

The wearable device is a portable device that can be directly worn by a user or integrated into clothes or accessories of the user. The wearable device in embodiments of this application may be a portable device that has a sensing function and an audio detection function.

In some embodiments of this application, the electronic device may further be a portable terminal device that further includes another function such as a personal digital assistant function and/or a music player function. An example embodiment of the portable terminal device includes but is not limited to a portable terminal device using iOS^{®}, Android^{®}, Microsoft^{®}, or another operating system. Alternatively, the portable terminal device may be another portable terminal device, for example, a laptop computer (laptop) with a touch-sensitive surface (for example, a touch panel). It should be further understood that in some other embodiments of this application, the electronic device may alternatively be a desktop computer with a touch-sensitive surface (for example, a touch panel), instead of the portable terminal device.

It should be understood that in embodiments of this application, "at least one" means one or more, and "a plurality of" means two or more. The term "and/or" describes an association relationship of associated objects, and indicates that three relationships may exist. For example, A and/or B may indicate the following three cases: Only A exists, both A and B exist, and only B exists, where A and B may be singular or plural. The character "/" generally indicates an "or" relationship between the associated objects. "At least one of the following" or a similar expression thereof indicates any combination of these items, including a single item or any combination of a plurality of items. For example, at least one of a, b, or c may indicate a, b, c, a and b, a and c, b and c, or a, b and c, where a, b, and c may be singular or plural.

A current method for detecting a respiratory disease based on a cough sound by a wearable device faces a problem of difficulty in identifying a source of the cough sound. Therefore, embodiments of this application provide an audio detection method and an electronic device. In this solution, an object that emits audio can be identified relatively accurately, thereby improving accuracy of identifying a source of the audio.

For example, the method provided in embodiments of this application may be applied to a wearable device. When the method provided in embodiments of this application is applied to the wearable device, the wearable device can accurately identify whether a detected cough sound belongs to a user wearing the wearable device, and can perform more accurate detection of a disease risk for the user based on the cough sound belonging to the user.

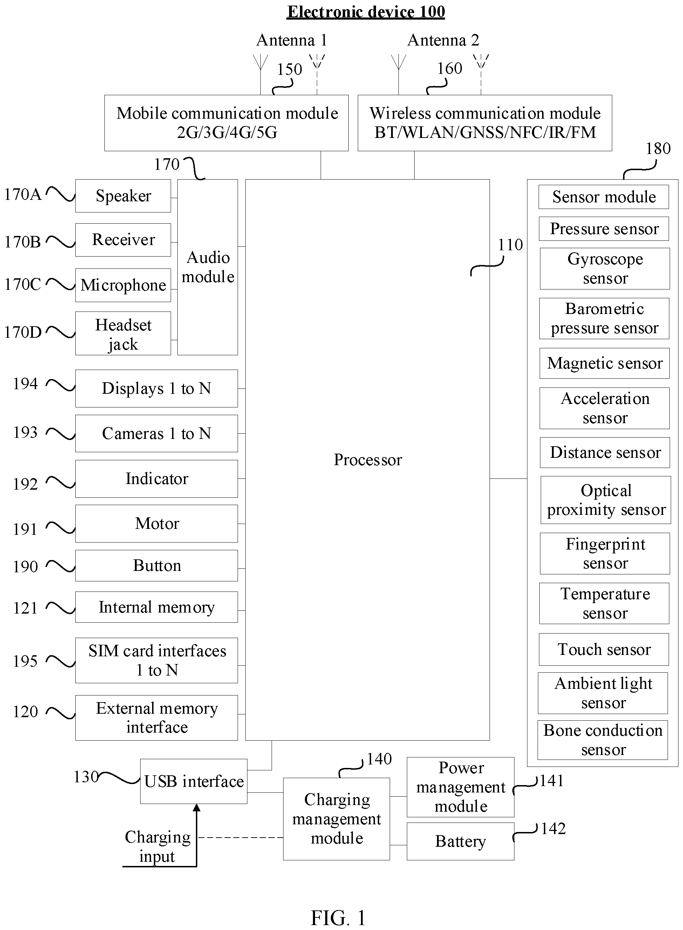

The following describes, with reference to FIG. 1, a structure of an electronic device to which a method provided in an embodiment of this application is applicable.

As shown in FIG. 1, the electronic device 100 may include a processor 110, an external memory interface 120, an internal memory 121, a USB interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a SIM card interface 195, and the like.

The sensor module 180 may include a gyroscope sensor, an acceleration sensor, an optical proximity sensor, a fingerprint sensor, a touch sensor, a temperature sensor, a pressure sensor, a distance sensor, a magnetic sensor, an ambient light sensor, a barometric pressure sensor, a bone conduction sensor, and the like.

It may be understood that the electronic device 100 shown in FIG. 1 is merely an example, and does not constitute a limitation on the electronic device. In addition, the electronic device may have more or fewer components than those shown in the figure, or may combine two or more components, or may have different component configurations. The various components shown in FIG. 1 may be implemented in hardware including one or more signal processing circuits and/or application-specific integrated circuits, software, or a combination of hardware and software.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (Neural-network Processing Unit, NPU), and/or the like. Different processing units may be independent components, or may be integrated into one or more processors. The controller may be a nerve center and a command center of the electronic device 100. The controller may generate an operation control signal based on instruction operation code and a time sequence signal, to complete control of instruction fetching and instruction execution.

A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor 110 may directly invoke the instructions or the data from the memory. This avoids repeated access, reduces waiting time of the processor 110, and improves system efficiency.

Execution of the audio detection method provided in embodiments of this application may be controlled by the processor 110 or implemented by invoking other components. For example, the processor 110 invokes a processing program stored in the internal memory 121 in this embodiment of this application, or invokes, by using the external memory interface 120, a processing program stored in a third-party device in this embodiment of this application, to control the wireless communication module 160 to perform data communication with another device, thereby improving intelligence and convenience of the electronic device 100 and improving user experience. The processor 110 may include different components. For example, when a CPU and a GPU are integrated, the CPU and the GPU may cooperate to perform the audio detection method provided in embodiments of this application. For example, in the audio detection method, some algorithms are performed by the CPU, and the other algorithms are performed by the GPU, to obtain relatively high processing efficiency.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1. The display 194 may be configured to display information entered by a user or information provided to a user, and various graphical user interfaces (graphical user interface, GUI). For example, the display 194 may display a photo, a video, a web page, a file, or the like.

In this embodiment of this application, the display 194 may be one integrated flexible display, or may be a spliced display including two rigid screens and one flexible screen located between the two rigid screens.

The camera 193 (a front-facing camera, a rear-facing camera, or a camera that may serve as both a front-facing camera and a rear-facing camera) is configured to capture a static image or a video. Usually, the camera 193 may include a photosensitive element such as a lens group and an image sensor. The lens group includes a plurality of lenses (convex lenses or concave lenses), and is configured to: acquire an optical signal reflected by a to-be-photographed object, and transfer the acquired optical signal to the image sensor. The image sensor generates an original image of the to-be-photographed object based on the optical signal.

The internal memory 121 may be configured to store computer-executable program code. The executable program code includes instructions. The processor 110 runs the instructions stored in the internal memory 121, to perform various function applications and data processing of the electronic device 100. The internal memory 121 may include a program storage area and a data storage area. The program storage area may store an operating system, code of an application (for example, an audio detection function), and the like. The data storage area may store data created during use of the electronic device 100, and the like.

The internal memory 121 may further store one or more computer programs corresponding to an audio detection algorithm provided in this embodiment of this application. The one or more computer programs are stored in the internal memory 121 and are configured to be executed by the one or more processors 110. The one or more computer programs include instructions, and the instructions may be used to perform steps in the following embodiments.

In addition, the internal memory 121 may include a high-speed random access memory, and may further include a non-volatile memory, for example, at least one magnetic disk storage device, a flash memory device, and a universal flash storage (universal flash storage, UFS).

Certainly, code of the audio detection algorithm provided in this embodiment of this application may alternatively be stored in an external memory. In this case, the processor 110 may run, by using the external memory interface 120, the code of the audio detection algorithm stored in the external memory.

The sensor module 180 may include a gyroscope sensor, an acceleration sensor, an optical proximity sensor, a fingerprint sensor, a touch sensor, and the like.

The touch sensor is also referred to as a "touch panel". The touch sensor may be disposed in the display 194, and the touch sensor and the display 194 form a touchscreen, which is also referred to as a "touch screen". The touch sensor is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor may be alternatively disposed on a surface of the electronic device 100, at a location different from that of the display 194.

For example, the display 194 of the electronic device 100 displays a home screen, and the home screen includes icons of a plurality of applications (for example, a camera application and a WeChat application). The user taps an icon of the camera application on the home screen by using the touch sensor, to trigger the processor 110 to start the camera application and turn on the camera 193. The display 194 displays an interface of the camera application, for example, a viewfinder interface.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide wireless communication solutions that are applied to the electronic device 100 and that include 2G/3G/4G/5G wireless communication solutions and the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules of the mobile communication module 150 may be disposed in the processor 110. In some embodiments, at least some functional modules of the mobile communication module 150 and at least some modules of the processor 110 may be disposed in a same device. In this embodiment of this application, the mobile communication module 150 may be further configured to exchange information with another device.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by using an audio apparatus (not limited to the speaker 170A, the receiver 170B, or the like), or displays an image or a video by using the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that is applied to the electronic device 100 and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processor module. The wireless communication module 160 receives an electromagnetic wave through the antenna 2, performs frequency modulation and filtering processing on the electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2. In this embodiment of this application, the wireless communication module 160 is configured to establish a connection to another electronic device, and exchange data. Alternatively, the wireless communication module 160 may be configured to access an access point device, send a control instruction to another electronic device, or receive data sent by another electronic device.

In addition, the electronic device 100 may implement an audio function, for example, music playing and recording, by using the audio module 170, the speaker 170A, the receiver 170B, the microphone 170C, the headset jack 170D, the application processor, and the like. The electronic device 100 may receive an input of the button 190, and generate a button signal input related to user setting and function control of the electronic device 100. The electronic device 100 may generate a vibration prompt (for example, an incoming call vibration prompt) by using the motor 191. The indicator 192 in the electronic device 100 may be an indicator light, may be configured to indicate a charging state and a battery level change, and may be further configured to indicate a message, a missed call, a notification, and the like. The SIM card interface 195 in the electronic device 100 is configured to connect to a SIM card. The SIM card may be inserted into the SIM card interface 195 or removed from the SIM card interface 195, to implement contact with or separation from the electronic device 100.

It should be understood that in actual application, the electronic device 100 may include more or fewer components than those shown in FIG. 1. This is not limited in this embodiment of this application. The electronic device 100 shown in the figure is merely an example, and the electronic device 100 may have more or fewer components than those shown in the figure, may combine two or more components, or may have different component configurations. The various components shown in the figure may be implemented in hardware including one or more signal processing circuits and/or application-specific integrated circuits, software, or a combination of hardware and software.

A software system of the electronic device 100 may use a layered architecture, an eventdriven architecture, a microkernel architecture, a micro service architecture, or a cloud architecture. In embodiments of this application, an Android system with a layered architecture is used as an example for describing a software structure of the electronic device.

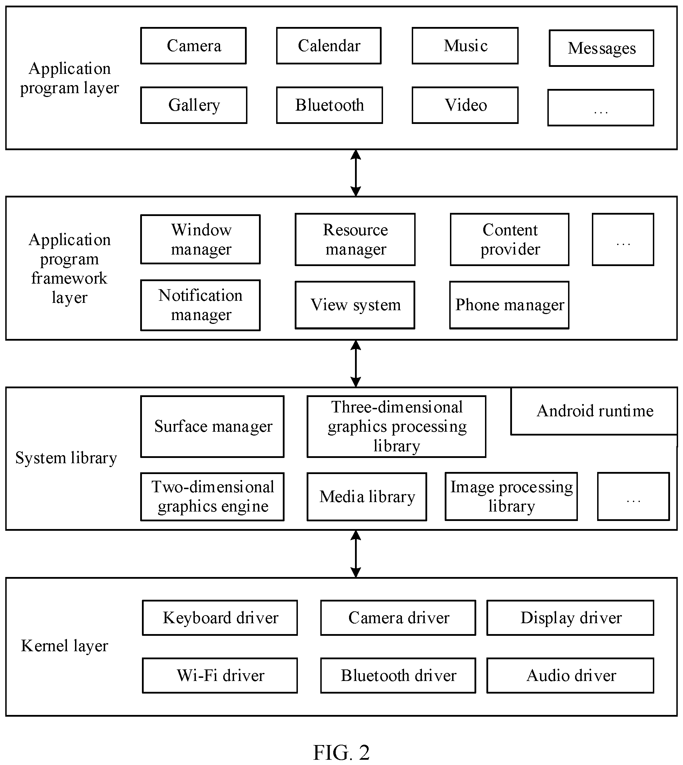

The layered architecture divides software into several layers, and each layer has a clear role and responsibility. The layers communicate with each other through a software interface. As shown in FIG. 2, the software architecture may be divided into four layers: an application program layer, an application program framework layer (framework, FWK), an Android runtime and system library, and a Linux kernel layer from top to bottom.

The application program layer is an uppermost layer of the operating system, and includes native applications of the operating system, such as Camera, Gallery, Calendar, Bluetooth, Music, Video, and Information. An application (application, APP) in embodiments of this application is a software program that can implement one or more specific functions. Usually, a plurality of applications, such as a camera application, a mailbox application, and a smart home device control application, may be installed in the electronic device. The application mentioned in the following may be a system application installed before delivery of the electronic device, or may be a third-party application downloaded from a network or obtained from another electronic device by a user in a process of using the electronic device.

Certainly, a developer may compile an application and install the application at the layer. In a possible implementation, the application may be developed by using a Java language, and is completed by invoking an application programming interface (application programming interface, API) provided by an application framework layer. A developer may interact with a bottom layer (for example, a kernel layer) of an operating system by using the application framework, to develop an application of the developer.

The application framework layer provides an API and a programming framework for an application at the application layer. The application framework layer may include some predefined functions. The application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to store and obtain data, and enable the data to be accessed by an application. The data may include information such as a file (for example, a document, a video, an image, or audio), and a text.

The view system includes visual controls, for example, a control for displaying content such as text, a picture, and a document. The view system may be used to construct an application. An interface in a display window may be formed by one or more views. For example, a display interface including a notification icon of Messages may include a view for displaying text and a view for displaying a picture.

The phone manager is configured to provide a communication function of the electronic device. The notification manager enables an application to display notification information in the status bar, and may be configured to convey a notification-type message. The displayed information may automatically disappear after a short pause without user interaction.

The Android runtime includes a core library and a virtual machine. The Android runtime is responsible for scheduling and management of the Android system.

The kernel library of the Android system includes two parts: one part is a function that needs to be invoked in a Java language, and the other part is the kernel library of the Android system. The application layer and the application framework layer run on the virtual machine. Java is used as an example. The virtual machine executes Java files at the application layer and the application framework layer as binary files. The virtual machine is configured to implement functions such as object lifecycle management, stack management, thread management, security and exception management, and garbage collection.

The system library may include a plurality of functional modules, for example, a surface manager, a media library, a three-dimensional graphics processing library (for example, OpenGL ES), and a two-dimensional graphics engine (for example, SGL). The surface manager is configured to manage a display subsystem and provide a fusion of two-dimensional and three-dimensional layers for multiple application programs. The media library supports playback and recording in a plurality of commonly used audio and video formats, and static image files. The media library may support a plurality of audio and video encoding formats such as MPEG-4, H.564, MP3, AAC, AMR, JPG, and PNG. The three-dimensional graphics processing library is configured to implement three-dimensional graphics drawing, image rendering, composition, layer processing, and the like. The two-dimensional graphics engine is a drawing engine for two-dimensional drawing.

The kernel (kernel) layer provides a core system service of the operating system. For example, security, memory management, process management, a network protocol stack, and a driver model are all based on the kernel layer. The kernel layer is also used as an abstraction layer between hardware and software stacks. The layer has many drivers related to the electronic device, and has the following main drivers: a display driver, a keyboard driver used as an input device, a flash driver based on a memory technology device, a camera driver, an audio driver, a Bluetooth driver, a Wi-Fi driver, and the like.

It should be understood that the foregoing function service is merely an example. In actual application, the electronic device may be divided into more or fewer function services based on another factor, or may be divided into functions of each service in another manner, or may not be divided into function services, but work as a whole.

The following describes in detail the solutions provided in this application with reference to specific embodiments.

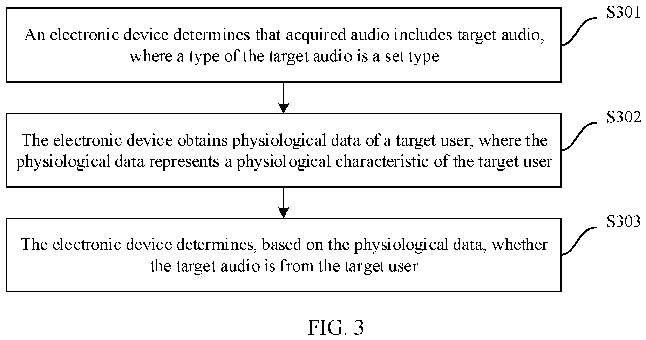

Refer to FIG. 3. An audio detection method provided in an embodiment of this application includes the following steps.

S301: An electronic device determines that acquired audio includes target audio, where a type of the target audio is a set type.

In some embodiments of this application, a target user may be a user wearing or carrying the electronic device, or the target user may be a preset user associated with the electronic device.

For example, the target audio (or the set type) may be a cough sound, a breath sound, a sneeze sound, a joint popping sound (namely, a sound made by a joint of a human body during movement), or the like.

In some embodiments of this application, the electronic device may be a device having an audio detection function. In this case, the electronic device can acquire and detect audio (or a sound) in real time in an environment in which the electronic device is located, to obtain a target audio signal (namely, an audio signal of the target audio). The electronic device may alternatively be a device having a wireless connection function. In this case, the electronic device can receive a target audio signal acquired by another audio detection device. Certainly, the electronic device may alternatively be a device having both the audio detection function and the wireless connection function. In this case, the electronic device can obtain the target audio signal in any one of the foregoing manners.

For example, the target audio may be a sound made by any user in the environment in which the electronic device is located. For example, when the electronic device and the target user are in a same environment, the electronic device may receive and identify, in real time, audio that appears in the environment. When identifying that the received audio is the set type, for example, the cough sound, the electronic device may determine that a user is coughing in the environment. Therefore, the electronic device may obtain an audio signal during coughing of the user, to obtain a corresponding cough sound signal.

In some embodiments of this application, after acquiring the target audio, the electronic device may identify, based on the target audio, a target action corresponding to the target audio. Specific identification may be implemented by using a trained network model.

In some embodiments of this application, after obtaining the target audio signal, the electronic device may first detect, based on the target audio signal, whether the user emitting the target audio has a disease risk. Specifically, after obtaining the target audio signal, the electronic device may first preprocess the target audio signal, for example, perform processing such as denoising, pre-emphasis, framing, and windowing. Then, characteristic data of the target audio may be extracted from the preprocessed target audio signal, and whether the user to which the target audio belongs has a disease risk is detected based on the extracted characteristic data.

The characteristic data of the target audio may include but is not limited to at least one of the following:

### (1) Time-domain characteristic

For example, the time-domain characteristic may be a zero-crossing rate (zero crossing rate, ZCR) of an audio signal. The zero-crossing rate is a quantity of times that a signal passes through a zero point (changes from positive to negative or from negative to positive) in each frame of the audio signal. The time-domain characteristic may further include a voice onset time, an autocorrelation parameter, a waveform characteristic of the audio signal, or the like. The voice onset time is a duration of audio energy in a rising phase. The autocorrelation parameter is a similarity between the audio signal and a time-shifted signal of the audio signal.

### (2) Frequency-domain characteristic

For example, the frequency-domain characteristic may be a Mel-frequency cepstral coefficient (Mel-frequency cepstral coefficient, MFCC), a power spectral density, a spectral centroid, a spectral flatness, a spectral flux, or the like. The MFCC is a cepstral coefficient extracted in the Mel-scale frequency domain, and the Mel scale describes nonlinear characteristics of frequency perceived by a human ear. The power spectral density is a power (mean-square value) per unit frequency band of a signal. The spectral centroid is a center of energy in a signal spectrum, and is used to describe brightness of a signal timbre. The spectrum flatness is a similarity between a quantized signal and noise. The spectral flux is a degree of variation between adjacent frames of the quantized signal.

### (3) Energy characteristic

For example, the energy characteristic may be root-mean-square energy or the like. The root-mean-square energy is average energy of a signal within a specific time range.

### (4) Musical characteristic

For example, the musical characteristic may be a fundamental frequency, an inharmonicity, or the like. The fundamental frequency is a frequency of a pitch of a sound. The inharmonicity is a degree to which an overtone frequency of a signal departs from integral multiples of a fundamental frequency.

### (5) Perception characteristic

For example, the perception characteristic may be sound loudness (intensity), sharpness, or the like. The loudness is signal strength (for example, a volume of a sound) sensed by the human ear. The sharpness indicates a magnitude of energy of a high frequency part in an audio signal. A larger energy of the high frequency part indicates a higher sharpness, and the sound sensed by the human ear is sharper.

In some embodiments of this application, the electronic device may detect, through model recognition, whether there is a disease risk based on the extracted characteristic data of the target audio. Specifically, after extracting the characteristic data of the target audio, the electronic device may input the extracted characteristic data into a trained disease screening model, to obtain a disease risk parameter output by the disease screening model. The parameter may indicate classification of the disease risk (for example, a high risk, a medium risk, or a low risk), or the parameter may indicate a specific value representing a magnitude of the disease risk. Optionally, the disease risk parameter may further indicate a disease type.

The disease screening model used by the electronic device may be obtained by training a network model using an algorithm, such as a logistic regression (logistic regression, LR) algorithm or an extreme gradient boosting (eXtreme Gradient Boosting, XGBoost) algorithm.

In some embodiments of this application, when detecting, based on the target audio signal, that the user emitting the target audio has a disease risk or a relatively high disease risk (for example, a case in which the disease risk parameter output by the disease screening model is greater than or equal to a set risk threshold), the electronic device may perform the following step S302 to obtain physiological data of the target user, and determine, based on the physiological data of the target user, whether the target audio is the audio emitted by the target user. Optionally, when detecting, based on the target audio signal, that the user emitting the target audio has no disease risk or has a relatively low disease risk (for example, a case in which the disease risk parameter output by the disease screening model is less than the set risk threshold), the electronic device may not perform additional processing, for example, only need to continue to perform original audio monitoring.

S302: The electronic device obtains the physiological data of the target user, where the physiological data represents a physiological characteristic of the target user.

In an actual scenario, a process of making a sound by the user is usually accompanied by execution of some actions. For example, when the user makes a cough sound, the user performs a coughing action, and when the user makes a breath sound, the user performs a breathing action. Therefore, there is a correspondence between the sound made by the user and the action performed by the user. That is, a type of the sound made by the user corresponds to a type of the action performed by the user. It may be considered that the sound made by the user is generated in a process of performing the action. Therefore, in this embodiment of this application, the target audio may be a sound made by the user to which the target audio belongs in a process of performing the target action. The target audio is the set type, and a type of the target action corresponds to the set type. For example, when the target audio is the cough sound, the breath sound, the sneeze sound, and the joint popping sound, corresponding actions are respectively coughing, breathing, sneezing, and a joint motion.

In addition, in a process of performing different actions by the user, physiological characteristics of the user change differently. Therefore, physiological characteristics of the user are usually different when the user performs different actions, and a corresponding action performed by the user may be identified based on the physiological characteristic of the user. Further, a sound made by the user may be associated with the physiological data of the user based on the action performed by the user, to associate the sound made by the user with the user. Therefore, in this embodiment of this application, the electronic device may identify, based on the physiological characteristic of the target user, an action performed by the target user, and then determine, based on a relationship between the action performed by the target user and an action corresponding to the audio detected by the electronic device, a relationship between the target audio detected by the electronic device and the target user.

In some embodiments of this application, the electronic device may be a device having a sensing function or a sensor. In this case, the electronic device can detect the physiological characteristic of the user in real time, and may acquire a physiological parameter representing the physiological characteristic of the user. The electronic device may alternatively be a device having a wireless connection function. In this case, the electronic device can receive physiological data acquired by another physiological monitoring device. Certainly, the electronic device may alternatively be a device having both the sensing function and the wireless connection function. In this case, the electronic device can obtain the physiological data of the user in any one of the foregoing manners.

In some embodiments of this application, in a process of acquiring the physiological data of the user or receiving the physiological data from the physiological monitoring device, the electronic device may store physiological data acquired in a time period closest to a current time. A duration of the time period may be a set duration.

For example, the electronic device may be a smartwatch or a mobile phone. When the electronic device is a smartwatch, the target user is a user wearing the smartwatch. The smartwatch may monitor the physiological characteristic of the target user, and acquire and store the physiological data of the user in real time. When the electronic device is a mobile phone, the target user may be a user holding the mobile phone or a registered owner of the mobile phone. The target user may wear a wearable device, for example, a band, used for physiological characteristic monitoring. In this case, the band may monitor the physiological characteristic of the target user in real time and send acquired physiological data to the mobile phone. The mobile phone may store the physiological data from the band. Optionally, after acquiring the physiological data, the band may not report the physiological data to the electronic device temporarily. Instead, the band first stores the acquired physiological data, and reports the physiological data according to an instruction of the electronic device when the electronic device needs the physiological data.

Based on the foregoing method, when performing step S302, the electronic device may obtain required physiological data from the physiological data stored in the electronic device, or may indicate the physiological monitoring device to report physiological data required by the electronic device.

In some embodiments of this application, the physiological data of the target user obtained by the electronic device is physiological data of the user acquired in a target time period. The target time period is a time period in which the electronic device or the audio detection device acquires the target audio signal, and is also a time period in which the target audio is generated. Based on this, it can be ensured that a generation time of the physiological data obtained by the electronic device is consistent with a generation time of the target audio, so that matching can be performed based on an audio signal and physiological data in a same time period, to ensure matching accuracy.

Various sensors may be configured in the electronic device or the physiological monitoring device to obtain physiological data through detection. In some embodiments of this application, the physiological data obtained by the electronic device includes at least a motion posture parameter and/or a heart rate parameter. The motion posture parameter indicates a motion or posture characteristic of a measured user, and may be measured by a device such as an acceleration sensor (acceleration transducer, ACC) or a gyroscope. The heart rate parameter indicates a heart rate and a pulse characteristic of the measured user, and may be obtained by a photoelectric sensor using photoplethysmography (photoplethysmography, PPG). Optionally, the physiological data may further include but is not limited to at least one of the following: a respiration rate parameter, a blood oxygen parameter, a blood pressure parameter, a pulse parameter, and the like. These parameters may be obtained through analysis and calculation on data measured by a sensor such as the ACC, the gyroscope, or the photoelectric sensor.

The physiological data may be used to detect an action performed by a corresponding user, to determine, based on whether the action performed by the user is consistent with the action corresponding to the target audio, whether the target audio belongs to the user. For example, when the motion posture parameter measured by the ACC has a relatively large peak value or a relatively significant fluctuation over time, and there is a significant increase in the heart rate parameter, it may be determined that the user performs a coughing action. If a target audio information signal is the cough sound signal, it may be determined that the target audio belongs to the user.

S303: The electronic device determines, based on the physiological data, whether the target audio is from the target user.

After obtaining the physiological data of the target user, the electronic device may determine, based on the physiological data, whether the target audio is from (belongs to) the target user. Specifically, the electronic device may identify, by using a pre-trained action recognition model and based on the physiological data of the target user, whether the target user performs the target action corresponding to the target audio, and then determine, based on whether the target user performs the target action, whether the target audio matches the target user. A type of the target action corresponds to a type of the target audio, and the target action may be determined based on the target audio. In some embodiments of this application, the action recognition model may be obtained by training a network model using an algorithm, such as a support vector machine (support vector machines, SVM) algorithm or an LR algorithm.

In an optional implementation, a physiological sample parameter used when the user performs various actions may be acquired in a model training phase, to form a model database of various physiological parameters. In a matching phase, the action recognition model may match physiological data in an input model with the physiological parameters in the model database. If a physiological parameter of a type of action has a highest matching degree with physiological data of an input action recognition model or the matching degree is greater than a set matching degree threshold, it is determined that this type of action is an action corresponding to the physiological data of the input action recognition model. If this type of action is the same as the target action, it may be determined that the user to which the physiological data belongs, namely, the target user, performs the target action, so that it may be determined that the target audio is a sound emitted by the target user in a process of performing the target action. Otherwise, if this type of action is different from the target action, a determination result that the target audio is not the sound emitted by the target user in a process of performing the target action may be obtained.

In another optional implementation, when source detection is performed on a specific type of audio, for example, when source detection is performed on audio when the target action is performed, only physiological sample parameters when different users perform the target action may be obtained in the model training phase, to form a model database corresponding to various physiological parameters. In the matching phase, the action recognition model may match a physiological parameter in the input model with the physiological parameters in the model database. If matching degrees between input physiological data and more than a set quantity of physiological parameters in the model database are all greater than the set matching degree threshold, it may be determined that the target audio is a sound emitted by the target user in a process of performing the target action. Otherwise, it is determined that the target audio is not a sound emitted by the target user in a process of performing the target action.

In still another optional implementation, the action recognition model may be configured to identify a user status, a user action posture, or the like corresponding to the input physiological data. When it is determined that an action performed by the user corresponding to the physiological data is the target action, it may also be determined that the target user to which the physiological data belongs performs the target action. In this case, it may be considered that the physiological data matches the target audio signal, so that it is determined that the target audio is a sound made by the target user in a process of performing the target action. In this manner, in the model training phase, a data pair may be formed by using physiological data acquired when the user performs different actions and a corresponding action indication label, and the data pair is used as training data, to train the network model.

In some embodiments of this application, input data of the action recognition model may be physiological data obtained by the electronic device, and output data of the action recognition model may be a determination result of whether the target user performs the target action, or may be a confidence level of the action recognition model. The confidence level indicates a possibility that the user performs the target action. A higher confidence level indicates a higher probability that the user performs the target action. The electronic device inputs obtained physiological data into the action recognition model, to obtain a recognition result that is output by the action recognition model and that is about whether the user performs the target action or a corresponding confidence level, thereby determining, based on the recognition result or the corresponding confidence level, whether the target audio is a sound made by the target user.

Specifically, when an output result is that the target user performs the target action or that the confidence level of the model is greater than or equal to a set first confidence level threshold, the electronic device may determine that the target audio is from the target user. When the output result is that the target user does not perform the target action or that the confidence level of the model is less than or equal to a set second confidence level threshold, the electronic device may determine that the target audio is not from the target user. When the output result is that the confidence level of the model is less than the first confidence level threshold and greater than the second confidence level threshold, the electronic device may further determine a relationship between the target audio and the target user by prompting the target user to perform confirmation. The first confidence level threshold is greater than the second confidence level threshold.

Optionally, when determining that the confidence level of the foregoing model is greater than or equal to a set third confidence level threshold, the electronic device may alternatively determine that the target audio is from the target user, and when determining that the confidence level of the model is less than the third confidence level threshold, the electronic device determines that the target audio is not from the target user.

When prompting the target user to perform confirmation, the electronic device may display prompt information to query whether the user has performed the target action, and determine, based on information fed back by the user, whether the user has performed the target action. If the user has performed the target action, the electronic device finally determines that the target audio is from the target user. Otherwise, the electronic device determines that the target audio is not from the target user.

For example, when the electronic device is a smartwatch and the target action is coughing, when prompting the target user to perform confirmation, the electronic device may prompt, in a manner such as vibration, the target user to view a display of the electronic device, and display an interface of prompt information on the display. The interface includes prompt information, for example, "Did you cough just now?", and further includes control options that can be operated by the user, for example, options of "Yes" and "No". The target user may tap a corresponding option based on an actual situation of whether the user has coughed. After receiving an operation of the user, the electronic device may determine a status of the target user.

When determining that the target audio is not from the target user, the electronic device determines that the target audio is from another user present in an environment around the target user.

In some embodiments of this application, when determining that the target audio is from the target user, the electronic device may further determine a disease risk (including a type of a disease and a corresponding risk magnitude) of the target user. When determining that the target audio is from the another user present in the environment around the target user, the electronic device may further determine a disease risk of the another user. When determining that the another user may have an infectious disease, the electronic device may provide a related prompt for the target user, so that the target user responds based on the prompt, thereby avoiding or reducing a possibility of contracting a disease from the environment.

The following uses only an example in which the electronic device determines a disease risk of the target user for description. A method used by the electronic device to determine the disease risk of the another user is the same as a method used by the electronic device to determine the disease risk of the target user. Details are not described in the following again.

In an optional implementation, when determining the disease risk of the target user, the electronic device may use the disease risk parameter detected based on the characteristic data of the target audio in step S301 as a disease risk parameter of the target user, to obtain the disease risk of the target user.

In another optional implementation, the electronic device may identify and analyze the disease risk of the target user with reference to more aspects of information. For example, the electronic device may identify a corresponding disease risk parameter based on at least one of the characteristic data of the target audio, personal information of the target user, and the physiological data of the target user by using the trained disease screening model, to obtain the disease risk of the target user.

For example, the characteristic data of the target audio may include at least one of the characteristics provided in the foregoing embodiment. The personal information of the target user may include information such as a gender, an age, a height, and a weight of the target user, and the information may be entered by the user into the electronic device in advance.

In still another optional implementation, the electronic device may select a corresponding risk level from preset correspondences between different characteristics and risk levels based on at least one type of characteristic data, and use the selected risk level as a risk level of the target user, to obtain the disease risk of the target user.

For example, the characteristic data may include the foregoing plurality of types of characteristic data, and may further include at least one of the following characteristic parameters: a parameter indicating a strength characteristic of the target audio; a quantity of times that the electronic device or the audio detection device detects the target audio signal within a preset duration; a risk level output by the disease screening model; and a duration of a target audio signal with a longest duration time in target audio signals detected by the electronic device or the audio detection device a plurality of times. Certainly, the at least one parameter may also be used as characteristic data of the audio, and is used in the foregoing process of identifying a disease risk by using the disease screening model.

In a possible case, when the electronic device determines that the target user has a disease risk or has a relatively high disease risk, the electronic device may prompt the target user that the target user has a disease risk, so that the target user further seeks medical treatment or the like in a timely manner.

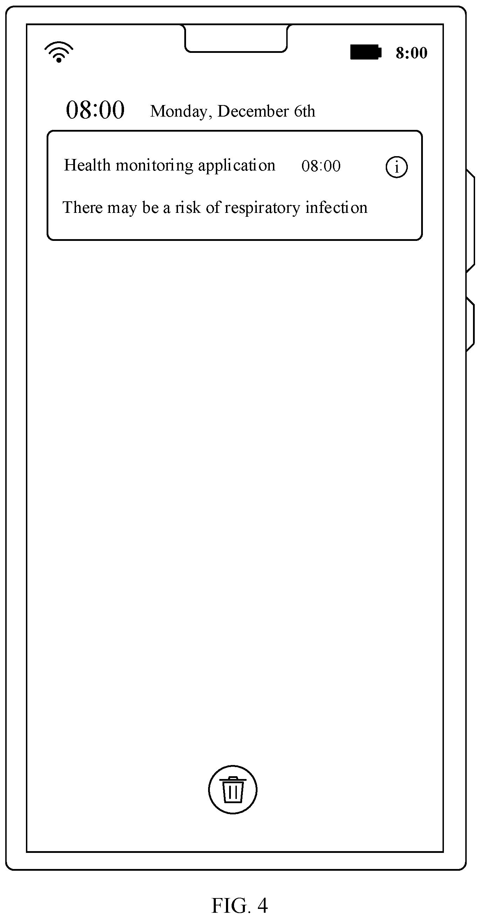

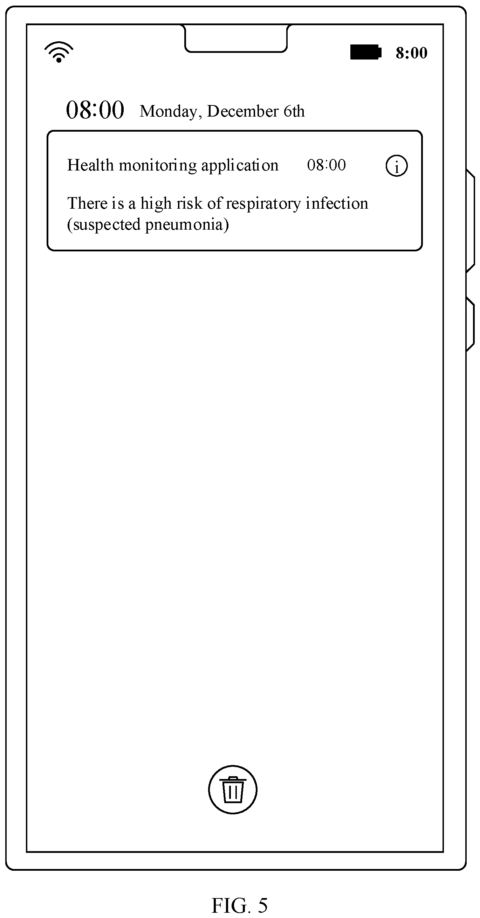

For example, when the electronic device is a mobile phone and the target action is coughing, the electronic device may prompt, in a manner such as a notification message prompt, the target user that there is a disease risk. For example, when the target user has a relatively low disease risk, the electronic device may display an interface shown in FIG. 4. When the target user has a relatively high disease risk, the electronic device may display an interface shown in FIG. 5. In the interface shown in FIG. 4 or FIG. 5, the electronic device may display risk prompt information (for example, "There may be a risk of respiratory infection" shown in FIG. 4 or "There is a high risk of respiratory infection (suspected pneumonia)" shown in FIG. 5) and may further display information such as a date and a time and a corresponding suggestion. For example, when determining that the user may have a risk of respiratory infection, the electronic device may further display the following suggestion information: Based on your recent detection data, you may have a risk of respiratory tract infection. Please take proactive measurement or seek medical attention for testing. When determining that the user has a high risk of respiratory infection, the electronic device may further display the following suggestion information: Based on your recent detection data, you may have a relatively high risk of respiratory tract infection. Please seek medical attention promptly. Optionally, the electronic device may further display prompt information such as "The detection should not be used as a basis for professional clinical diagnosis of respiratory health".

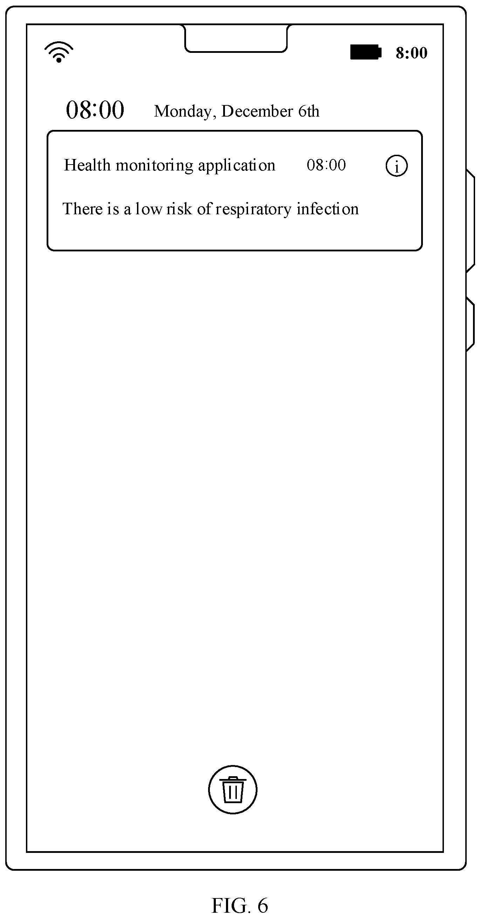

Optionally, when the electronic device detects, based on the target audio signal, that the user emitting the target audio has no disease risk or has a very low disease risk, the electronic device may also periodically display notification information to notify the user of a health status. For example, the electronic device may periodically display an interface shown in FIG. 6, to notify the user of a current disease risk, and may further display corresponding suggestions and prompt information such as "Through analysis of your recent measurement data, your risk of respiratory tract infection is low and falls within a healthy range. Keeping good life habits will bring you sustained health", and "The detection should not be used as a basis for professional clinical diagnosis of respiratory health".

In another possible case, when the electronic device determines that the another user has a disease risk or has a relatively high disease risk, and a disease type that the another user may suffer from is an infectious disease, the electronic device may prompt the target user that there may be a risk of disease infection in a surrounding environment, and prompt the target user to perform protection and the like.

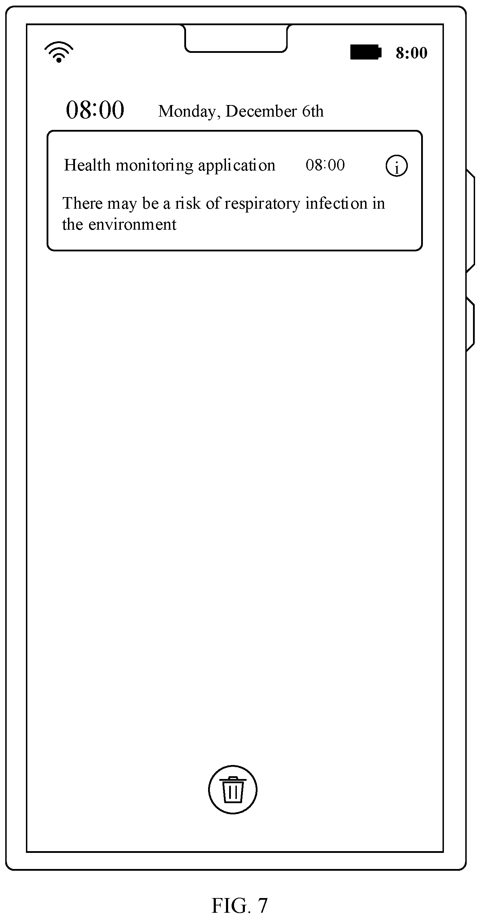

For example, when the electronic device is a mobile phone and the target action is coughing, the electronic device may prompt, in a manner such as a notification message prompt, the target user that there may be a risk of respiratory infection in a current environment. For example, the electronic device may display an interface shown in FIG. 7, and the interface may include information for prompting a risk level of respiratory infection in the environment. Optionally, the interface may further display a current location of the target user, a detected related characteristic parameter (for example, a quantity of cough sounds detected in the environment), a corresponding suggestion, and the like. For example, the interface may further include prompt information such as "You are currently located at X. A quantity of times of coughing in a surrounding environment within XX time is XXX, and it is recommended that you leave this place or take protective measures", and "The detection should not be used as a basis for professional clinical diagnosis of respiratory health".

Optionally, the electronic device may further display information such as a waveform of a detected cough sound on the display. This is not specifically limited in this embodiment of this application.

In the foregoing embodiment, the electronic device may determine a relationship between the target audio and the target user based on the physiological parameter of the target user when the target audio is detected, to distinguish audio of the target user from audio of the another user. Further, in a subsequent processing process, the target audio may also be processed based on a source of the target audio. For example, if it is determined that the source of the target audio is the target user, the target audio has a relatively high reference significance for detecting the disease risk of the target user, and a result detected based on the target audio signal also has relatively high reliability. Therefore, a disease status of the target user may be detected based on the target audio. If it is determined that the source of the target audio is not the target user, the target audio has relatively low reference significance for detecting the disease risk of the target user. This can avoid mistakenly using the target audio as the audio of the target user for disease risk detection, thereby improving accuracy of related detection.

The following describes the solutions provided in embodiments of this application by using an example in which the solutions provided in embodiments of this application are applied to a scenario of detecting a risk of respiratory tract infection of a user.

The following provides description by using an example in which the electronic device is a wearable device worn by a target user, and the target user detects, by using the wearable device, whether there is a risk of respiratory tract infection. The target audio is a cough sound. The wearable device has an audio detection function, and can detect a cough sound existing in an environment in which the wearable device is located, and detect a risk of respiratory tract infection based on the detected cough sound. In addition, the wearable device has a physiological monitoring function, and can monitor a physiological characteristic of the target user in real time.

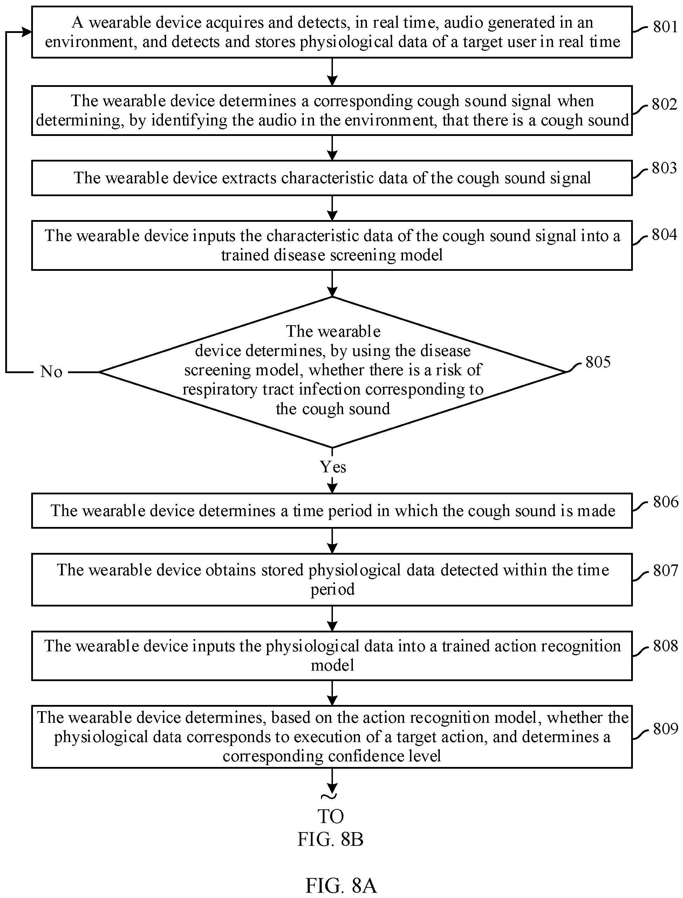

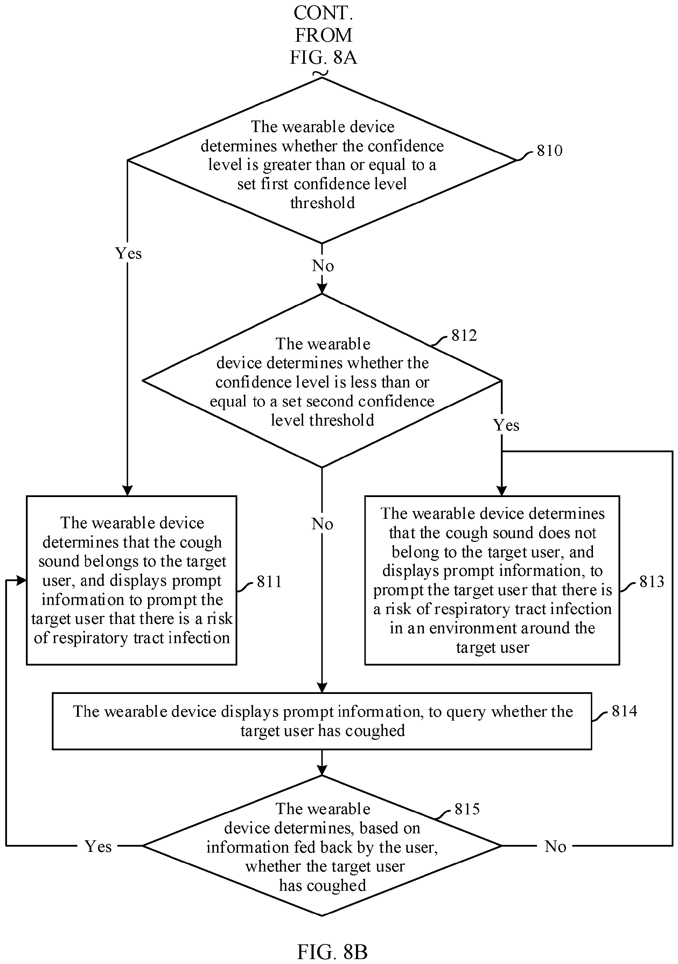

Refer to FIG. 8A and FIG. 8B. A procedure of a method for detecting a risk of respiratory tract infection of a user according to an embodiment of this application includes the following steps.

S801: The wearable device acquires and detects, in real time, audio generated in an environment, and detects and stores physiological data of the target user in real time.

A function of the wearable device to detect a risk of respiratory tract infection of the user may be controlled and implemented through a health monitoring application installed in the wearable device. After a respiratory tract monitoring function in the health monitoring application of the wearable device is enabled, the wearable device may perform the method provided in this instance to perform processing such as detection of a risk of respiratory tract infection. After the respiratory tract monitoring function in the health monitoring application of the wearable device is disabled, the wearable device may stop performing the method provided in this instance to perform processing such as detection of a risk of respiratory tract infection.

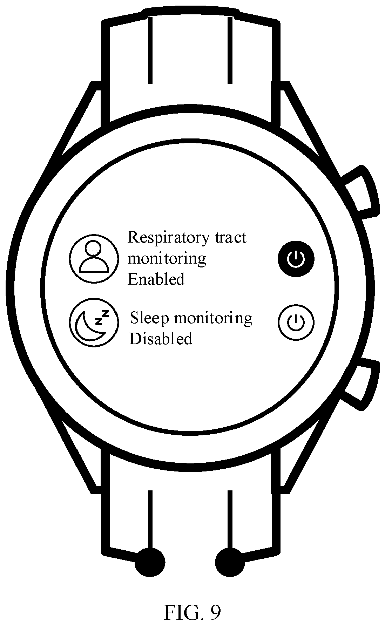

For example, as shown in FIG. 9, a control switch of a respiratory tract monitoring function may be provided in the health monitoring application of the wearable device. The user may control, by operating the control switch, to enable or disable the function of monitoring respiratory tract health. After the function of monitoring respiratory tract health is enabled, the wearable device may perform the method provided in this embodiment of this application, to monitor respiratory tract health of the user wearing the wearable device.

Optionally, after the user enables the function of monitoring respiratory tract health, the wearable device may run the function in the background, and an interface displayed in the foreground of the wearable device may be updated with a user operation.

S802: The wearable device determines a corresponding cough sound signal when determining, by identifying the audio in the environment, that there is a cough sound.

Optionally, the wearable device may identify, by using a trained audio recognition model, whether the acquired audio is a cough sound.

S803: The wearable device extracts characteristic data of the cough sound signal.

S804: The wearable device inputs the characteristic data of the cough sound signal into a trained disease screening model.

S805: The wearable device determines, by using the disease screening model, whether there is a risk of respiratory tract infection corresponding to the cough sound; and if there is a risk of respiratory tract infection corresponding to the cough sound, perform step S806; or otherwise, perform step S801.

For specific implementations of the foregoing step, refer to the method for detecting whether there is a disease risk by using the disease screening model in step S301. Details are not described herein again.

S806: The wearable device determines a time period in which the cough sound is made.

The time period includes at least a start time and an end time of the cough sound, and may further include a duration of the cough sound.

S807: The wearable device obtains stored physiological data detected within the time period.

For specific implementations of this step, refer to the related method in step S302. Details are not described herein again.

S808: The wearable device inputs the physiological data into a trained action recognition model.

S809: The wearable device determines, based on the action recognition model, whether the physiological data corresponds to execution of a target action, and determines a corresponding confidence level.

For specific implementations of this step, refer to the method for identifying the physiological data by using the action recognition model in step S303. Details are not described herein again.

S810: The wearable device determines whether the confidence level is greater than or equal to a set first confidence level threshold; and if the confidence level is greater than or equal to the set first confidence level threshold, perform step S811; or otherwise, perform step S812.

S811: The wearable device determines that the cough sound belongs to the target user, and displays prompt information to prompt the target user that there is a risk of respiratory tract infection.

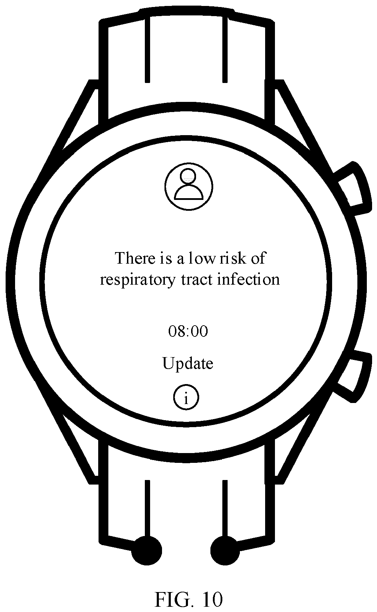

For example, the wearable device may display an interface shown in FIG. 10, and the interface includes prompt information prompting the target user that there is a risk of respiratory tract infection.

S812: The wearable device determines whether the confidence level is less than or equal to a set second confidence level threshold; and if the confidence level is less than or equal to the set second confidence level threshold, perform step S813; or otherwise, perform step S814.

The second confidence level threshold is less than the first confidence level threshold.

S813: The wearable device determines that the cough sound does not belong to the target user, and displays prompt information, to prompt the target user that there is a risk of respiratory tract infection in an environment around the target user.

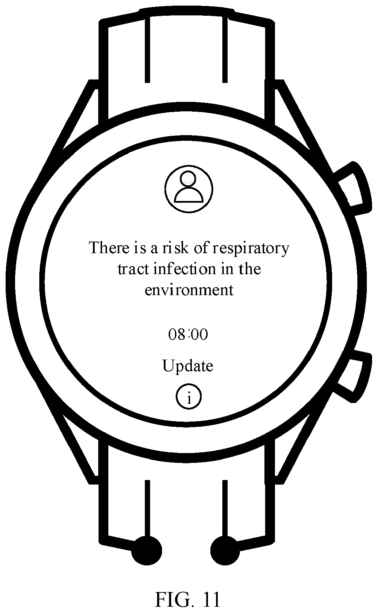

For example, the wearable device may display an interface shown in FIG. 11, and the interface includes prompt information prompting the target user that there is a risk of respiratory tract infection in the environment around the target user.

S814: The wearable device displays prompt information, to query whether the target user has coughed, and performs step S815.

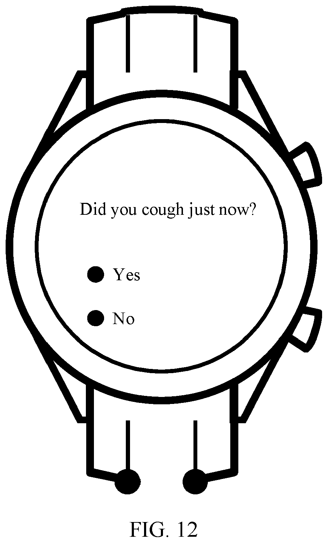

For example, the wearable device may display an interface shown in FIG. 12, and the interface includes prompt information querying whether the user has coughed.

S815: The wearable device determines, based on information fed back by the user, whether the target user has coughed; and if the target user has coughed, perform step S811; or otherwise, perform step S813.

For specific execution of the foregoing steps, refer to related descriptions in the foregoing embodiment. Details are not described again in this instance.

It should be noted that specific implementation procedures provided in the foregoing instances are merely examples of the method procedures applicable to embodiments of this application. A performing sequence of the steps may be correspondingly adjusted based on an actual requirement, or another step may be added, or some steps may be reduced.

In the foregoing embodiment, by detecting the source of the cough sound, the wearable device can ensure that a cough sound for predicting a respiratory disease risk is a sound from the target user, thereby reducing interference caused by an incorrectly acquired audio signal to a prediction process, and further improving prediction accuracy. In addition, by distinguishing whether the source of the cough sound is the target user wearing the wearable device or another user in the environment, a risk of respiratory tract infection of the target user and a risk of respiratory tract infection in the environment may be separately predicted. This can improve acquiring effect and prediction effect of the cough sound of the target user. In addition, an infection source and the risk in the environment are determined, so that the target user can be prompted in time to take measures such as personal protection to reduce the infection risk. In conclusion, this solution can quickly identify the source of the cough sound, and further predict and warn a risk of respiratory tract infection. Therefore, real-time performance and implementation costs are relatively low, and user experience can be greatly improved. In addition, this solution has great application value in a public scenario such as an indoor scenario, and has high feasibility.

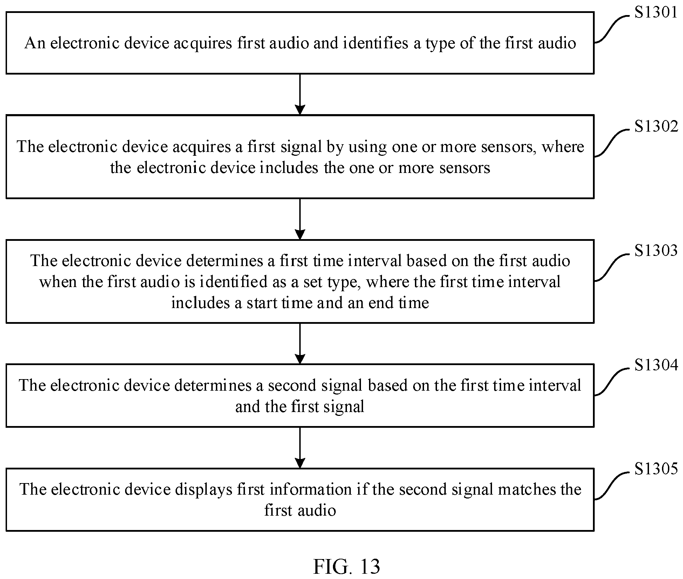

Based on the foregoing embodiments and a same concept, an embodiment of this application further provides an audio detection method. As shown in FIG. 13, the method includes the following steps.

S1301: An electronic device acquires first audio and identifies a type of the first audio.

For example, the electronic device may be the electronic device in the foregoing embodiment (FIG. 3), or may be the wearable device in the foregoing embodiment (FIG. 8A and FIG. 8B).

S1302: The electronic device acquires a first signal by using one or more sensors, where the electronic device includes the one or more sensors.

The first signal is a signal acquired for the first user. For example, the first user may be the target user in the foregoing embodiment. The first signal may be a signal corresponding to the physiological data in the foregoing embodiment, and the electronic device may determine the physiological data in the foregoing embodiment based on the acquired first signal.

S1303: The electronic device determines a first time interval based on the first audio when the first audio is identified as a set type, where the first time interval includes a start time and an end time.

For example, the first audio of the set type may be the target audio in the foregoing embodiment, and the first time interval is a time interval in which the first audio is generated, and may be the target time period in the foregoing embodiment.

S1304: The electronic device determines a second signal based on the first time interval and the first signal.

For example, the second signal may be a signal corresponding to the physiological data in the target time period in the foregoing embodiment, and the electronic device may determine the physiological data based on the acquired second signal.

S1305: The electronic device displays first information if the second signal matches the first audio.

Optionally, the electronic device displays second information if the second signal does not match the first audio.

For example, the first information may be the notification information for notifying the target user that there is a disease risk in the foregoing embodiment, for example, the information shown in FIG. 6 or FIG. 10. The first information may be the notification information for notifying that there is an infection risk in the environment around the target user in the foregoing embodiment, for example, the information shown in FIG. 7 or FIG. 11.

Specifically, for specific steps performed by the electronic device in the method, refer to related descriptions in the foregoing embodiments. Details are not described herein again.

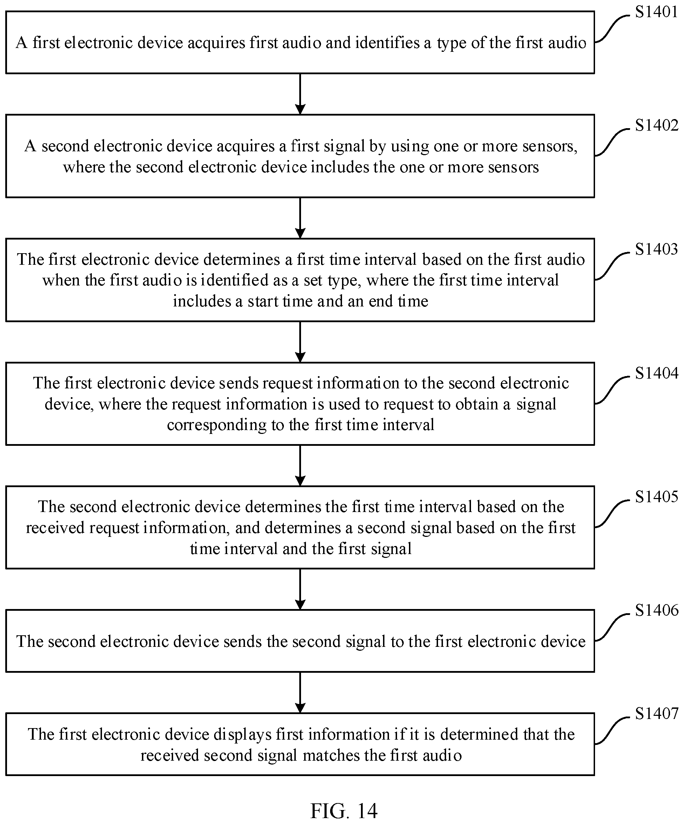

Based on the foregoing embodiments and a same concept, an embodiment of this application further provides an audio detection method, applied to a system including a first electronic device and a second electronic device. As shown in FIG. 14, the method includes the following steps.

S1401: The first electronic device acquires first audio and identifies a type of the first audio.

For example, the first electronic device may be the electronic device in the foregoing embodiment (FIG. 3). For example, the first electronic device may be a mobile terminal device such as a mobile phone.

S1402: The second electronic device acquires a first signal by using one or more sensors, where the second electronic device includes the one or more sensors.

For example, the second electronic device may be the physiological monitoring device in the foregoing embodiment. For example, the second electronic device may be a wearable device such as a watch or a band.

The first signal is a signal acquired for the first user. For example, the first user may be the target user in the foregoing embodiment. The first signal may be a signal corresponding to the physiological data in the foregoing embodiment.

It should be noted that there is no strict time sequence limitation on performing step S1401 and step S1402. For example, step S1401 may be performed earlier than step S1402 or may be performed later than step S1402, or step S1401 and step S1402 may be performed simultaneously (or synchronously).

S1403: The first electronic device determines a first time interval based on the first audio when the first audio is identified as a set type, where the first time interval includes a start time and an end time.

For example, the first audio of the set type may be the target audio in the foregoing embodiment, and the first time interval is a time interval in which the first audio is generated, and may be the target time period in the foregoing embodiment.

S1404: The first electronic device sends request information to the second electronic device, where the request information is used to request to obtain a signal corresponding to the first time interval.

S1405: The second electronic device determines the first time interval based on the received request information, and determines a second signal based on the first time interval and the first signal.

For example, the second signal may be a signal corresponding to the physiological data in the target time period in the foregoing embodiment.

S1406: The second electronic device sends the second signal to the first electronic device.

S1407: The first electronic device displays first information if it is determined that the received second signal matches the first audio.

Optionally, the electronic device displays second information if the second signal does not match the first audio.

For example, the first information may be the notification information for notifying the target user that there is a disease risk in the foregoing embodiment, for example, the information shown in FIG. 6 or FIG. 10. The first information may be the notification information for notifying that there is an infection risk in the environment around the target user in the foregoing embodiment, for example, the information shown in FIG. 7 or FIG. 11.

Specifically, for specific steps performed by the first electronic device or the second electronic device in the method, refer to related descriptions in the foregoing embodiments. Details are not described herein again.

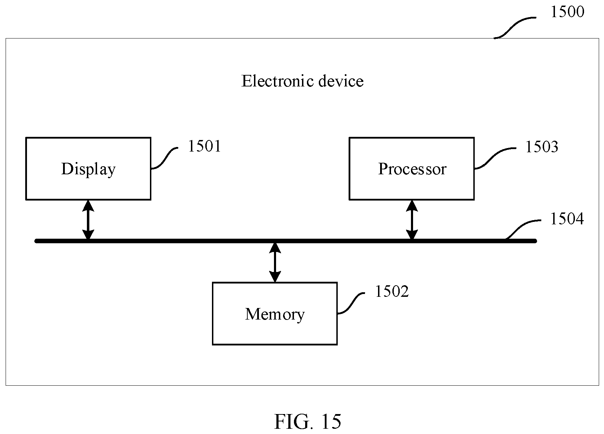

Based on the foregoing embodiments and a same concept, an embodiment of this application further provides an electronic device. The electronic device is configured to implement the audio detection method provided in embodiments of this application. As shown in FIG. 15, the electronic device 1500 may include a display 1501, a memory 1502, one or more processors 1503, and one or more computer programs (not shown in the figure). The foregoing components may be coupled by using one or more communication buses 1504.

The display 1501 is configured to display a related user interface such as an image, a video, or an application interface. The memory 1502 stores one or more computer programs (code). The one or more computer programs include computer instructions. The one or more processors 1503 invoke the computer instructions stored in the memory 1502, so that the electronic device 1500 performs the audio detection method provided in embodiments of this application.

During specific implementation, the memory 1502 may include a high-speed random access memory, and may also include a non-volatile memory such as one or more magnetic disk storage devices, a flash device, or another non-volatile solid-state storage device. The memory 1502 may store an operating system (briefly referred to as a system below), for example, an embedded operating system such as Android, iOS, Windows, or Linux. The memory 1502 may be configured to store an implementation program in embodiments of this application. The memory 1502 may further store a network communication program. The network communication program may be used to communicate with one or more additional devices, one or more user equipments, or one or more network devices. The one or more processors 1503 may be a general-purpose central processing unit (Central Processing Unit, CPU), a microprocessor, an application-specific integrated circuit (Application-Specific Integrated Circuit, ASIC), or one or more integrated circuits configured to control program execution in the solutions of this application.

It should be noted that FIG. 15 is merely an implementation of the electronic device 1500 provided in this embodiment of this application. In actual application, the electronic device 1500 may alternatively include more or fewer components. This is not limited herein.

Based on the foregoing embodiments and a same concept, an embodiment of this application further provides a system. The system includes the first electronic device and the second electronic device.

Based on the foregoing embodiments and a same concept, an embodiment of this application further provides a computer-readable storage medium. The computer-readable storage medium stores a computer program. When the computer program is run on a computer, the computer is enabled to perform the method provided in the foregoing embodiments of this application.

Based on the foregoing embodiments and a same concept, an embodiment of this application further provides a computer program product. The computer program product includes a computer program or instructions. When the computer program or the instructions are run on a computer, the computer is enabled to perform the method provided in the foregoing embodiments of this application.

All or some of the methods in embodiments of this application may be implemented by using software, hardware, firmware, or any combination thereof. When software is used to implement embodiments, the foregoing embodiments may be implemented completely or partially in a form of a computer program product. The computer program product includes one or more computer instructions. When the computer program instructions are loaded and executed on the computer, the procedures or functions according to embodiments of the present invention are all or partially generated. The computer may be a general-purpose computer, a dedicated computer, a computer network, a network device, a user equipment, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (digital subscriber line, DSL for short)) or wireless (for example, infrared, radio, or microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a digital video disc (digital video disc, DVD for short)), a semiconductor medium (for example, an SSD), or the like.

It is clear that a person skilled in the art can make various modifications and variations to this application without departing from the scope of this application. In this way, if these modifications and variations of this application fall within the scope of the claims of this application and equivalent technologies thereof, this application is also intended to include these modifications and variations.

## Claims

1. An audio detection method, applied to an electronic device, wherein the method comprises:
acquiring first audio, and identifying a type of the first audio;
acquiring a first signal by using one or more sensors, wherein the electronic device comprises the one or more sensors;
determining a first time interval based on the first audio when the first audio is identified as a set type, wherein the first time interval comprises a start time and an end time;
determining a second signal based on the first time interval and the first signal; and
displaying first information if the second signal matches the first audio.

2. The method according to claim 1, wherein the method further comprises:
displaying second information if the second signal does not match the first audio.

3. The method according to claim 2, wherein before the determining a first time interval based on the first audio, the method further comprises:
determining a first risk parameter based on the first audio, wherein the first risk parameter is greater than or equal to a set threshold, and the first risk parameter indicates a disease risk corresponding to the first audio.

4. The method according to claim 3, wherein
the first information indicates that the first audio is from a first user and/or that the first user has a disease risk, wherein the first user is a user wearing the electronic device; and
the second information indicates that the first audio is not from the first user and/or that there is an infection risk in an environment around the first user.

5. The method according to claim 3 or 4, wherein the determining a first risk parameter based on the first audio comprises:
extracting characteristic data from the first audio, wherein the characteristic data represents a time-domain characteristic and/or a frequency-domain characteristic of the first audio; and
determining the first risk parameter based on a set disease screening model and the characteristic data, wherein the disease screening model indicates a relationship between a time-domain characteristic and/or a frequency-domain characteristic of audio and a risk parameter corresponding to the audio.

6. The method according to any one of claims 1 to 5, wherein before the displaying first information if the second signal matches the first audio, the method further comprises: determining, based on the first audio, a first action corresponding to the first audio, and determining, based on the second signal, a second action corresponding to the second signal; and
that the second signal matches the first audio comprises: a type of the second action corresponding to the second signal is the same as a type of the first action corresponding to the first audio.

7. The method according to claim 6, wherein the determining, based on the second signal, a second action corresponding to the second signal comprises:
determining the second action and a confidence level of the second action based on a set action recognition model and the second signal, wherein the action recognition model represents a relationship between the second signal and both the second action and the confidence level, and the confidence level represents recognition accuracy of the action recognition model; and
determining that the confidence level is greater than or equal to a set first threshold; or
determining that the confidence level is greater than or equal to a set second threshold and is less than or equal to a set third threshold; displaying first indication information, wherein the first indication information indicates to confirm whether the second action is performed; and receiving second indication information, wherein the second indication information indicates a confirmation that the second action is performed.

8. The method according to any one of claims 1 to 7, wherein the second signal comprises at least one of the following:
a signal acquired by using an acceleration sensor;
a signal acquired by using a gyroscope sensor; and
a signal acquired by using a photoelectric sensor.

9. The method according to any one of claims 1 to 8, wherein the set type comprises at least one of the following:
a cough sound, a breath sound, a sneeze sound, and a joint popping sound.

10. An audio detection method, applied to a first electronic device, wherein the method comprises:
acquiring first audio, and identifying a type of the first audio;
determining a first time interval based on the first audio when the first audio is identified as a set type, wherein the first time interval comprises a start time and an end time;
sending request information to a second electronic device, wherein the request information is used to request to obtain a signal corresponding to the first time interval;
receiving a first signal from the second electronic device, wherein the first signal is acquired by the second electronic device by using one or more sensors, wherein the second electronic device comprises the one or more sensors; and
displaying first information if the first signal matches the first audio.

11. The method according to claim 10, wherein the method further comprises:
displaying second information if the first signal does not match the first audio.

12. The method according to claim 11, wherein before the determining a first time interval based on the first audio, the method further comprises:
determining a first risk parameter based on the first audio, wherein the first risk parameter is greater than or equal to a set threshold, and the first risk parameter indicates a disease risk corresponding to the first audio.

13. The method according to claim 12, wherein
the first information indicates that the first audio is from a first user and/or that the first user has a disease risk, wherein the first user is a user wearing the second electronic device; and
the second information indicates that the first audio is not from the first user and/or that there is an infection risk in an environment around the first user.

14. The method according to claim 12 or 13, wherein the determining a first risk parameter based on the first audio comprises:
extracting characteristic data from the first audio, wherein the characteristic data represents a time-domain characteristic and/or a frequency-domain characteristic of the first audio; and
determining the first risk parameter based on a set disease screening model and the characteristic data, wherein the disease screening model indicates a relationship between a time-domain characteristic and/or a frequency-domain characteristic of audio and a risk parameter corresponding to the audio.

15. The method according to any one of claims 10 to 14, wherein before the displaying first information if the first signal matches the first audio, the method further comprises: determining, based on the first audio, a first action corresponding to the first audio, and determining, based on the first signal, a second action corresponding to the first signal; and
that the first signal matches the first audio comprises: a type of the second action corresponding to the first signal is the same as a type of the first action corresponding to the first audio.

16. The method according to claim 15, wherein the determining, based on the first signal, a second action corresponding to the first signal comprises:
determining the second action and a confidence level of the second action based on a set action recognition model and the first signal, wherein the action recognition model represents a relationship between the first signal and both the second action and the confidence level, and the confidence level represents recognition accuracy of the action recognition model; and
determining that the confidence level is greater than or equal to a set first threshold; or
determining that the confidence level is greater than or equal to a set second threshold and is less than or equal to a set third threshold; displaying first indication information, wherein the first indication information indicates to confirm whether the second action is performed; and receiving second indication information, wherein the second indication information indicates a confirmation that the second action is performed.

17. The method according to any one of claims 10 to 16, wherein the first signal comprises at least one of the following:
a signal acquired by using an acceleration sensor;
a signal acquired by using a gyroscope sensor; and
a signal acquired by using a photoelectric sensor.

18. The method according to any one of claims 10 to 17, wherein the set type comprises at least one of the following:
a cough sound, a breath sound, a sneeze sound, and a joint popping sound.

19. An electronic device, comprising a display, a memory, and one or more processors, wherein
the memory is configured to store computer program code, and the computer program code comprises computer instructions; and when the computer instructions are executed by the one or more processors, the electronic device is enabled to perform the method according to any one of claims 1 to 9, or perform the method according to any one of claims 10 to 18.

20. A computer-readable storage medium, wherein the computer-readable storage medium stores a computer program, and when the computer program is run on a computer, the computer is enabled to perform the method according to any one of claims 1 to 9, or perform the method according to any one of claims 10 to 18.

21. A computer program product, wherein the computer program product comprises a computer program or instructions, and when the computer program or the instructions are run on a computer, the computer is enabled to perform the method according to any one of claims 1 to 9, or perform the method according to any one of claims 10 to 18.
